# EUROPEAN PATENT APPLICATION

(11) **EP 0 995 404 A1**
(43) Date of publication of application: **26.04.2000**
(21) Application number: 98500228.6
(22) Date of filing: 20.10.1998
(51) Int. Cl.: A61B 17/70

(54) **Cervical plate**

(71) Applicant: INDUSTRIAS QUIRURGICAS DE LEVANTE, 46988 Paterna - Valencia (ES)
(72) Inventor: Sebastian Bueno, César, 46988 Paterna-Valencia (ES); De Gracia Castillo de Olivares, Javier, 46988 Paterna-Valencia (ES)
(74) Representative: Urizar Anasagasti, José Antonio

(57) **Abstract**

The present invention refers to a cervical plate made of titanium alloy to be mounted anteriorly, having a curved profile in longitudinal section and in transverse section, provided with a spicule (4) on each corner, said spicules designed to penetrate the anterior vertebral cortex, and also incorporating some holes (2) at the plate (1) corners and centre (3), having a tapered seat (9) to receive the screws (5) in such a way that the screw can take a sloped position when tightened, specially indicated for front discal rupture, fractures, tumours, cervical distonies, cervical spondylarthrosis, canal stenosis, etc. It can also be applied to the anterior fastening of cervical osseous inserts.

## Description

The present invention relates to a cervical plate used to integrate and fasten an osseous insert in a base formed on the cervical vertebrae through forward approach, by using some spicules provided on the insert forward corners to be nailed into the vertebrae when the plate fastening screws are screwed in, said screws possibly being directed as required, and also provided with intermediate conical holes used for fastening the insert.

The inserts known to be attached onto cervical vertebrae can be introduced either forwardly or backwardly, said inserts being used depending of the different applications what implues different techniques, instruments and implants. With respect to the known implants to be introduced forwardly, they consist of plates placed on the insert, being fastened to the best condition neighbour vertebrae.

This attachement is usually made with screws, by means of peripherical screws introduced into or threaded onto said neighbour vertebrae to the insert, fastening the implant to the vertebral column and others are screwed into same insert by fastening it to the plate, thus making an integrated assembly.

One of the inconveniences of these plates is the possible loosening and lack of attachement, due to the fact that cervical vertebrae are in continuous movement so that the vertebrae anchoring screws expand the hole diameter, losing the definite attachement and allowing the insert to be expelled out of the hole.

In order to overcome this loosening problem the plate used to be fastened by means of bicortical screws, since these screws, in contrast with those described, pass through the first vertebra cortical, the spongious area and at last a second cortical of the vertebra opposite surface, screwing into back corticals of vertebrae bodies, thus providing a more solid anchoring to obtain a less realessable assembly. However, this procedure implieas a serious risk of introducing the screws two turns in excess through the back cortical which can produce a medular compression with a subsequent neurological damage to the patient.

Another system used consists of utilizing plates provided with screws having upper portion and grooved head, so that after the screw is fastened to the vertebra body (without passing through the ooposite cortical), a smaller srew is inserted acting as a expander screw in the main screw grooved head, this head being compressed against the plate hole.

The main inconvenience in this system consists of the risk that all those screwing operations must be made during the surgery, wherein the operation area is rather small, and there exists the risk of loosing or dropping the small expander screw or loosening same with the subsequent complete unfastening of plate.

Another procedure,with a higher or lower difficulty is that in which as a first operation some threaded stems will be fastened to the vertebrae body, said stems having an upper end with a smaller diameter threaded surface placed outside the vertebrae cortical. Further, the stem heads are introduced though the plate holes, remaining exposed and then the plate is fastened to said stems by means of small flat nuts which attach the plate to the screw.

The main problem in this system is the use of small size components such as nuts, involving the use of a larger amount of more precise and sensitive tools, making the implant process more difficult. Also, it presents the inconvenience that the screws must be mounted on the vertebrae by means of a jig so that any minimal error of placement or orientation will prevent from a correct introduction of the plate onto the stems heads, causing an implant serious problem for the system does not accept any clearance..

Also, fully flat cervical plates are used during surgery, and said plates must be conformed depending on their positioning on the vertebrae. This procedure enjoys an easier manufacturing process, but complcates the surgical operation for it requires special tools and procedures to obtain the required shape.

Another system uses expanding bolts which, by inserting another small screw inside said expanding bolt, this bolt opens, thus providing an attachement to the vertebrae spongious. However this procedure has the inconvenience of destroying a great amount of spongious tissue, for it must be of smaller diameter than other screws and making it difficult to extract same if required, since the expanded bolt is filled with bone so that the extraction of said bolt is blocked inside.

The use of the cervical plate, as the object of present invention, intends to overcome all above mentioned inconveniences, and to obtain first an undamaged back cortical a second a perfect condition of blocking the interphase plate-vertebrae, for it practically eliminates the micro movement, often of shearing character, produced between the own plate and the vertebral body surface for it may start the bolt loosening when said bolt swings within the vertebral body spongious.

It is based on a cervical plate applied from the front side, said plate being provided with some spicules oriented perpendicular to the corners of the plate plan. Said spicules being introduced into the forward cortical of vertebral body on tightening the fastening screws. Furthermore, the plate holes matching said screws are flat and slightly bent toward the corners with a bevel for a correct seating of tapered head crews.

In the other hand the screws have a rough exterior finish to have a good adherence to the spongious bone and a perfect osteointegration, all system components being made in a titanium alloy (Ti6A14V) with mechanical and structural features such that they provide a high biological tolerance, allowing for compatibility when carrying out further magnetic resonance studies.

The plate is formed and molded to provide a morphology with bends on the side area as well as on the cross section, so that it can be adapted properly to the front surface of vertebral bodies and recover the phisiological cervical lordosis, thus preventin the plate conforming during the surgery.

The spicules have a sharp and hard point which can be easily introduced into the body, obtaining a complete attachment from the first moment of tightening, since once the spicules are placed on the bone the plate is totally blocked, and on tightening the screws the spicules fully penetrate so that there will be four anchorage points making it difficult any plate movement during the process.

In this manner a perfect anchorage of the plate-vertebra interphase is obtained preventing any relative displacement for the spicules do not allow the relative movement of a part with rescpect to the other, preventing to a great extent the bone wear due to friction of the crew inside the matching hole. Due to this fact, it is not required to make biocortical implants since monocortical implants will be sufficient, as they provide a good coupling between vertebra and implant avoinding the inconveniences of biocortical implants.

The plate holes are striped or grooved, i.e., they allow for screw placement not only in several positions along the hole but also the screw can take a sloped position when being tightened. This permits an easier positioning of these plates and screwing same to the bone with a wide tolerance with respect to orientation and screw penetration, providing a great flexibility to the implant.

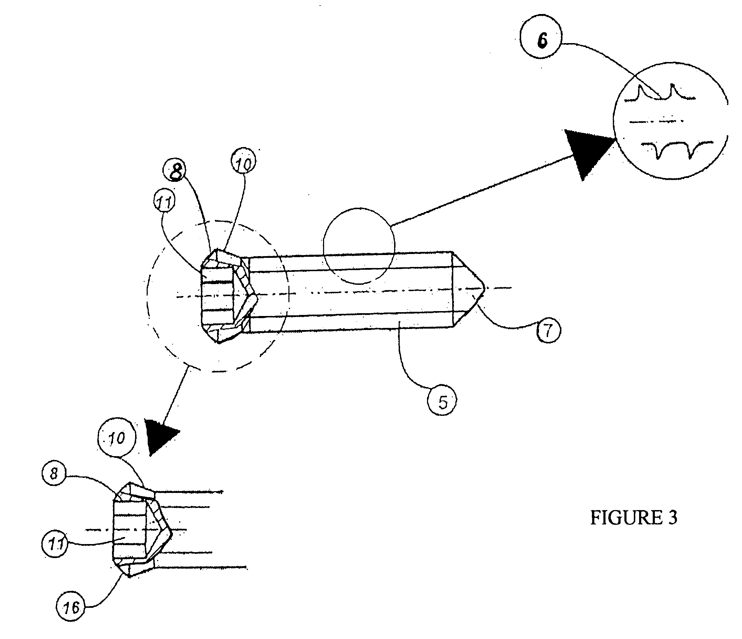

In order to better understand this description and being part of the same, a set of figures is included in order to illustrate, without any limiting effects, the following:
- Figure 1 -: Plan view of the plate and a detail of a corner with enlarged spicule;
- Figure 2 -: Details of the plate curved profile
A - Convex longitudinal curve of plate
B - Concave cross curve profile of plate
- Figure 3 -: Front view of the fastening screw with details of screw head and stem

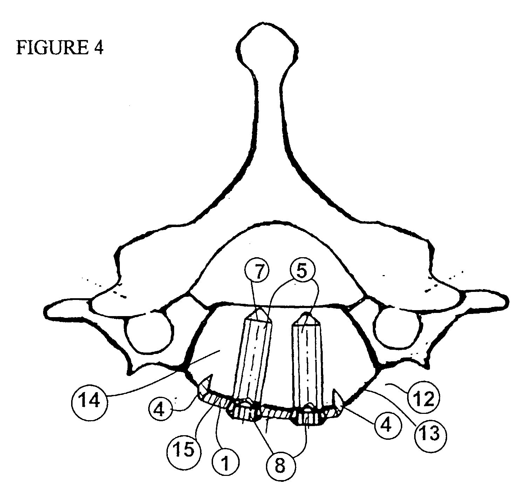

- Figure 4 -: Section of a plate in implant position

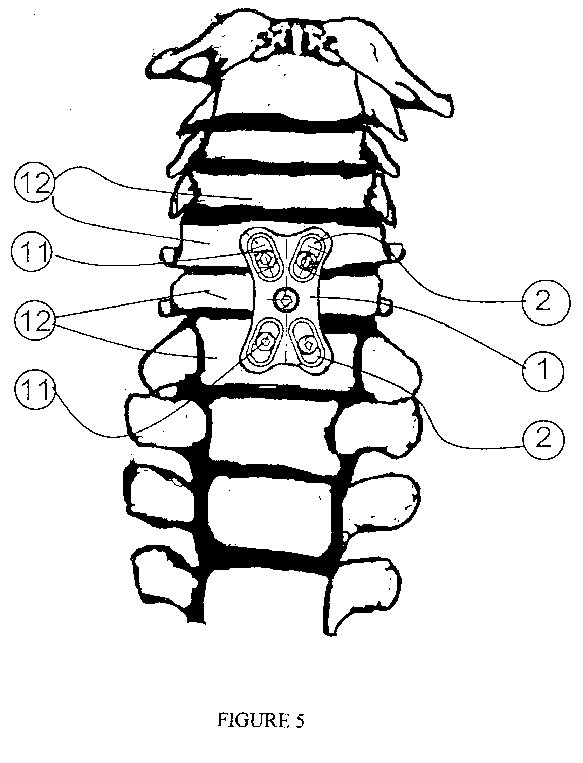

- Figure 5 -: Plan view of the plate object of present invention implanted on cervical

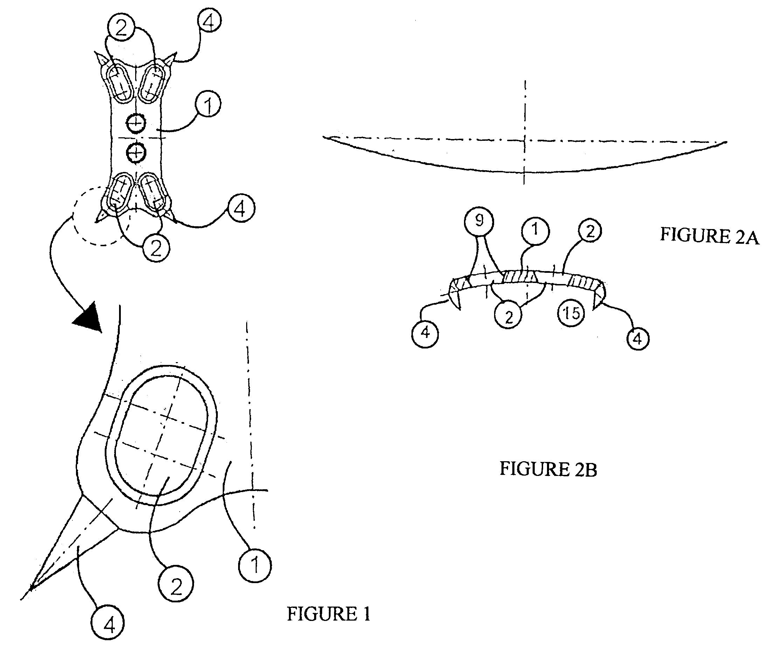

As it can be seen in the figures, there is a plate body (1), having some striped or grooved holes (2), some other central holes (3), the spicules or pins (4) curved as shown on the drawings of figure 2. In the other hand, the fastening screw comprising a bolt (5) with rough surface (6), with tapered end (7) and tapered side (10) head (8), as well as the holes (2) sides (9), said head housing an allen type hexagonal cavity (11). Said plate is placed on two vertebrae (12), which comprise a cortical zone (13) surrounding the spongious (14).

The application of said plate is simple, and to do it, once the implant has been placed in the vertebrae (12), the plate (1) is placed above same and the points corresponding to drilled holes, which will house matching screws, will be marked on the adjacent vertebrae. First a drill will be introduced in the vertebral body, following a threading tool corresponding to th selected screw, making said holes without removing the plate (1).

The spicule points (4), nail shaped, start penetrating into the cortical bone on progressively tightening the corner screws (5), becoming totally fastened when the head (8) with its tapered sides (10) exerts a pressure over the plate holes (2) sides (9).

Since these holes (2) are grooved or striped, the screw (5) can be placed in any allowed position, and also said screw can take some sloping in order to match the hole drilled in the vertebrae, for the screw head (8) sides (10) tapering is long enough and matching the hole (2) walls (9), so that the setting and coupling between head and hole will be precise.

When tightening these screws (5), the point (7) penetrates in the vertebra, through the cortical (13) or hard zone, and is being housed in the spongious (14) or soft zone. In order to increase the contact surface of screws (5) with the spongious, in order to provide a better tightening, the stem (5) will have a rough surface (6).

Furthermore, the system applied for tightening the screws is the head recess (8) allen type (11), so that an adequate tool will be used, with no need to bite the screw head exterior surface or to use a screwdriver which very often will damage the head groove.

The pressure exerted by the screw (5) head (6) on the plate (1), through the contact surfaces of the hole (2) sides (9), will force said plate to move towards the vertebral body (12), with the spicules penetrating progressively inti the cortical hard zone. This tightening or setting operation ends when the plate (1) bottom surface (15) gets in contact with the vertebra cortical (13). At that moment the spicule will be fully introduced with the maximum tightening.

The curved cross section profile of said plate (1) provides a wide contact surface with the vertebrae (12), since that curved profile corresponds to the anatomical profile. Thus, making it easier the coupling and fastening of the plate to the vertebrae, locging the insert and preventing any relative displacement to each other. Also, the plate longitudinal curve profile is designed to be fully adapted to front surface of vertebral bodies (12) and to recover the cervical phisiological lordosis.

The screw head (8), has a rounded flat finish (16) on its upper portion with a low profile, with the purpose of not damaging any soft areas or producing scabs in gullet or orogullet. Said holes (3) are to house the screws fastening the insert to the plate (1).

Once the nature of the present invention, as well as an embodiment of same, have been described suficiently, we must only add that it will be possible to introduce changes in shape, materials and application of same, as long as those alterations do not substantially affect to the characteristics of the invention as claimed below.

## Claims

1. Cervical plate to be mounted through front side, characterized in that it comprises a plate body (1), having some grooved or striped holes (2), some other central holes (3), the spicules (4) having longitudinal a cross section curved profiles, wherein the fastening screw comprises a rough surface (6) stem (5) with a tapered tip (7) and rounded surface (16) head (8) having tapered sides (10), said head (8) having an allen type hexagonal recess (11).

2. Cervical plate according to the first claim, characterized in that the plate (1) has a concave cross section curved profile with similar radius to the vertebra anatomical curvature (12), as well as a convex curve profile along the longitudinal area.

3. Cervical plate according to the first claim, characterized in that the spicules (4) have a tapered sharp, hard tip, like a nail, with said spicules tipspointing toward the vertebrae body (12).

4. Cervical plate, according to the first claim, characterized in that the striped or grooved holes (2) have a tapered interior wall (9), with a slope matching the fastening screw (5) head tapered sides (10)
